# EUROPÄISCHE PATENTSCHRIFT

(11) **EP 2 731 582 B1**
(45) Veröffentlichungstag und Bekanntmachung des Hinweises auf die Patenterteilung: **30.01.2019**
(21) Anmeldenummer: 12705681.0
(22) Anmeldetag: 24.02.2012
(51) Int. Cl.: A61K 8/41, A61K 8/42, A61K 8/55, A61K 8/60, A61K 8/73, A61Q 17/04, A61K 8/11, A61K 8/14

(54) **LADUNGSGEBER FÜR EIN VESIKULÄRES TRÄGERSYSTEM EINES UV-SCHUTZMITTELS FÜR DIE HAUT ODER DIE HAARE**
CHARGE PROVIDERS FOR A VESICULAR CARRIER SYSTEM OF A UV PROTECTION COMPOSITION FOR THE SKIN OR HAIR
TRANSMETTEUR DE CHARGE POUR SYSTÈME EXCIPIENT VÉSICULAIRE D'UN AGENT DE PROTECTION CONTRE LES RAYONS ULTRAVIOLETS, POUR LA PEAU OU LES CHEVEUX

(30) Priorität: 19.05.2011 DE 102011076149
(43) Veröffentlichungstag der Anmeldung: 21.05.2014
(73) Patentinhaber: Evonik Schlüchtern GmbH, 36381 Schlüchtern (DE)
(72) Erfinder: BEYER, Monika, 61130 Nidderau-Ostheim (DE); SACHER, Michael, 36381 Schlüchtern (DE); TEICHMÜLLER, Dirk, 63589 Linsengericht (DE); TEICHMÜLLER, Sarah, 63589 Linsengericht (DE)
(74) Vertreter: Sommer, Andrea
(86) Internationale Anmeldenummer: PCT/EP2012/053136
(87) Internationale Veröffentlichungsnummer: WO 2012/156109

(56) Entgegenhaltungen:
- WO-A1-01/62376
- WO-A1-2010/092142
- DE-A1-102010 030 000
- US-A1- 2008 317 836
- Monika Beyer: "UV-Nanocapsules - Performance enhancement of organic UV-filters through nanoencapsulation", , März 2010 (2010-03), XP002719381, Gefunden im Internet: URL:http://www.rovicosmetics.de/rovi2005/u pload/publikationen/BEYER_SunCare20101.pdf [gefunden am 2014-01-29]
- "Liposome", Wikipedia , 21 February 2016 (2016-02-21), Retrieved from the Internet: URL:https://en.wikipedia.org/wiki/Liposome
- A. WAGNER ET AL.: "Liposome Technology for Industrial Purposes", JOURNAL OF DRUG DELIVERY, vol. 2011, 591325, 2011, pages 1-9,
- Dr. Hans Lautenschläger: "Handbook of Cosmetic Science and Technology", 2006 pages 155-163,
- "Handbook of detergents, Part D: Formulation", 2006 vol. 128, pages 221-223,
- "Handbook of detergents: Part E: Applications", 2009 vol. 141, pages 359-360,

## Beschreibung

Die vorliegende Erfindung betrifft eine kosmetische oder pharmazeutische Formulierung nach Anspruch 1. Das für das menschliche Auge sichtbare Sonnenlicht erstreckt sich über ein Strahlungsspektrum von 400 bis 800 nm. Unterhalb von 400 nm liegt das Spektrum der UV-Strahlung (UV=Ultraviolett). Obwohl die Ultraviolettstrahlung die niederenergetischste ionisierende Strahlungsform darstellt, kann sie für dieser Strahlung exponierte Körperoberflächen schädlich sein.

Auf der Haut bewirken UV-A-Strahlen (320 bis 400 nm) u.a. eine Schädigung der Kollagene, wodurch die Haut an Spannkraft verliert und daher frühzeitig altern kann. Außerdem führt erhöhte Strahlenbelastung im UV-A-Bereich zu einem höheren Melanomrisiko. UV-B-Strahlen (280 bis 320 nm) führen zu entzündungsbedingter Hautrötung und verursachen den allseits bekannten Sonnenbrand.

Für die Haare geht mit der UV-Bestrahlung ein photochemisch induzierter Verlust von Proteinen und der Abbau des Haarfarbstoffs Melanin einher. UV-B-Strahlen sorgen dabei vor allem für morphologische Schäden am Haar, wie z.B. den Abbau von Haarproteinen, während UV-A-Strahlen besonders biochemische Veränderungen und Farbveränderungen verursachen können. Aminosäuren, aus denen die Haarproteine zusammengesetzt sind, sind lichtempfindlich. Ihre photochemische Schädigung produziert freie Radikale, die ihrerseits die Proteinstruktur des Keratins und damit die Haare weiter schädigen.

Es besteht daher ein Bedarf nach UV-Schutzmitteln, um sich gegen die vorgenannten schädlichen Wirkungen des UV-Anteils im Sonnenlicht zu schützen. Solche UV-Schutzmittel werden üblicherweise auf die Haut oder auf die Haare aufgetragen, um die negativen Wirkungen der Sonnenstrahlung (wie Sonnenbrand, Hautalterung, Schädigung des Haares) zu verhindern. Hierfür enthalten die genannten UV-Schutzmittel geeignete UV-Filtersubstanzen.

Bei den UV-Filtersubstanzen wird zwischen chemischen und physikalischen UV-Filtersubstanzen unterschieden. Chemische Filtersubstanzen absorbieren energiereiche Strahlung und geben sie als energieärmere, langwelligere Strahlung oder Wärme wieder ab. Physikalische Filtersubstanzen dagegen streuen und reflektieren das Licht hauptsächlich. Da die einzelnen Substanzen in der Regel keinen Schutz über das gesamte UV-Spektrum hinweg bieten, werden meist mehrere Stoffe kombiniert.

Um einen optimalen UV-Schutz zu erlangen, muss eine möglichst hohe UV-Filtersubstanzkonzentration in einem UV-Schutzmittel vorliegen. Dies ist bei vielen herkömmlichen UV-Schutzmitteln nur bedingt in dem gewünschten Maße zufriedenstellend gewährleistet. Insbesondere werden die für einen optimalen UV-Schutz gewünschten Konzentrationen häufig dann nicht erreicht, wenn eine Kombination von zwei oder mehr verschiedenen UV-Filtersubstanzen mit synergistischem Wirkspektrum in jeweils hoher Konzentration erforderlich ist, um ein UV-Schutzmittel mit einem optimalen Breitbandschutz über verschiedene Bereiche des relevanten UV-Spektrums zu erreichen

Die Veröffentlichung "UV-Nanocapsules, Performance enhancement of organic UV-filters through nanoencapsulation" ("Focus on SUN CARE", Beilage zu "Household and Personal Care TODAY", n. 3/2010) beschreibt UV-Nanokapseln mit einer mittleren Partikelgröße von 100-300 nm, bei denen das Innere öllösliche UVA- und UVB-Filter enthält und die Hülle aus hydrophobierten Polysacchariden gebildet wird. Der Zusatz eines Coemulgators sorgt dafür, dass die Hülle positiv geladen ist.

Der vorliegenden Erfindung liegt daher die Aufgabe zugrunde, ein UV-Schutzmittel bereitzustellen, in dem UV-Filtersubstanz in sehr hohen Konzentrationen enthalten sein kann. Insbesondere soll es möglich sein zwei oder mehr verschiedene UV-Filtersubstanzen mit synergistischem Wirkspektrum in jeweils sehr hoher Konzentration in das UV-Schutzmittel stabil einzubringen, um ein UV-Schutzmittel mit einem optimalen Breitbandschutz über verschiedene Bereiche des relevanten UV-Spektrums zu erreichen. Desweiteren soll die Möglichkeit bestehen, Stoffe, die in der Lage sind, UV-Filtersubstanzen vor Zersetzung durch UV-Strahlung zu schützen und sie somit zu stabilisieren, sogenannte Photostabilisatoren, zusammen mit den UV-Filtersubstanzen in das UV-Schutzmittel stabil zu integrieren.

Darüber hinaus ist es gewünscht, dass das UV-Schutzmittel mit den vorgenannten Fähigkeiten auch eine gute Adhäsion an Haut und Haar bietet, um damit eine möglichst lange Verweildauer der UV-Filtersubstanz bzw. der UV-Filtersubstanzen auf der Haut oder den Haaren zu gewährleisten. Insbesondere soll die Adhäsion so effektiv sein, dass die UV-Filtersubstanz bzw. die UV-Filtersubstanzen auch mehrere Waschgänge auf der Haut bzw. den Haaren überdauern kann. Diese Aufgabe wird erfindungsgemäß gelöst durch ein UV-Schutzmittel der eingangs genannten Art, wobei die Vesikel aus hydrophobierten Polysacchariden eine Teilchengröße von 10 bis 1000 nm sowie eine positive Oberflächenladung mit einem Zetapotential in dem Bereich von 1 bis 150 mV aufweisen, und wobei die positive Oberflächenladung dadurch bewirkt wird, dass die Vesikel zusätzlich zu den Polysacchariden, aus denen die Vesikel aufgebaut sind, positiv geladene Moleküle als Ladungsgeber aufweisen, wobei diese Ladungsgeber unter einem oder mehreren der Folgenden Ladungsgeber a) bis f) ausgewählt sind:

### a) N-Stearyl-N,N-dimethyl-N-(2-hydroxy-3-panthenyl)propyl-ammoniumsalze mit der Formel (I),

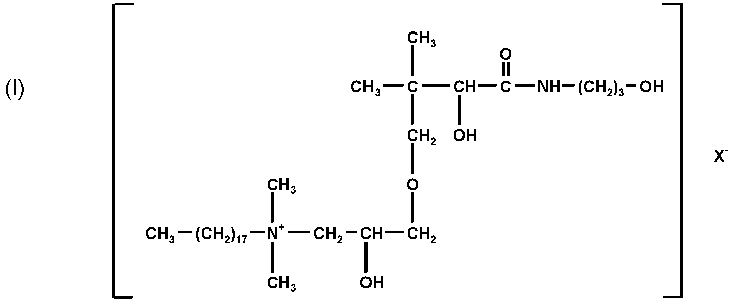

wobei X⁻ in den oben angegebenen Formeln (I) ein kosmetisch oder pharmazeutisch verträgliches organisches oder anorganisches Anion ist. Vorzugsweise ist X⁻ ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, lodid, Saccharinat, Tosylat oder Methosulfat, ganz besonders bevorzugt Chlorid.

### b) Positiv geladene quaternäre Zuckerderivate ("Zuckerquats") mit der Formel (II)

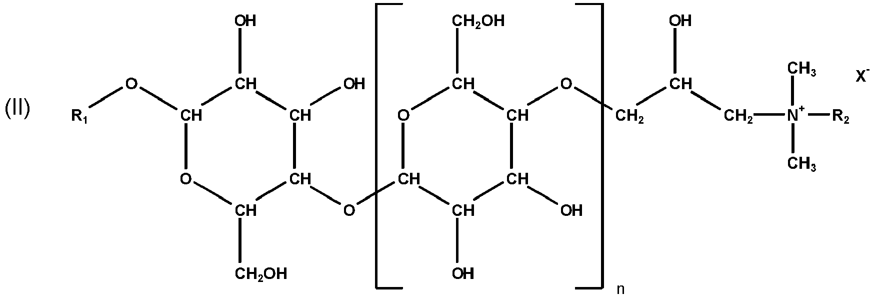

Vorzugsweise verfügen die quaternären Zuckerderivate ("Zuckerquats") über Monosaccharideinheiten die 6 Kohlenstoffatome aufweisen. Besonders bevorzugt sind die Zuckerquats ausgewählt unter Glucose, Fructose, Mannose und/oder Galactose. Bei besonders bevorzugten Ausführungsformen liegen die Monosaccharide als Disaccharideinheiten vor. Insbesondere sind Zuckerquats bevorzugt, bei denen die Monosaccharide die Disaccharideinheiten Maltose und/oder Lactose bilden.

Bevorzugt handelt es sich bei den quaternären Zuckerderivaten um Etherverbindungen von Alkyl-Glucopyranosiden (n=0) und/oder Alkyl-Glucopyranosyl-glucopyranosiden (n=1) mit N-Alkyl-N,N-Dimethyl-N-(2,3-Dihydroxypropyl)-ammonium-salzen und Kombinationen davon. Der an die Zuckereinheit gebundene Alkylrest R₁ ist bevorzugt eine unverzweigte oder verzweigte Alkylkette mit C₄-C₁₄ und weist besonders bevorzugt C₄, C₁₀ und/oder C₁₂ Kohlenstoffatome auf. Der an das quaternäre Stickstoffatom gebundene Alkylrest R₂ ist bevorzugt eine Methylgruppe oder ganz bevorzugt eine unverzweigte oder verzweigte Alkylkette mit C₄-C₁₈ und weist besonders bevorzugt C₄, C₁₂ und/oder C₁₈ Kohlenstoffatome auf.

Bei den vorgenannten Etherverbindungen kann es sich um einheitliche Substanzen handeln. In der Regel werden jedoch zur Herstellung dieser Stoffe vornehmlich natürliche Öle, bevorzugt Kokosöl und Palmöl/Palmkernöl herangezogen, so dass neben den primär genannten unverzweigten oder verzweigten Alkylresten R₁ und R₂ entsprechend dem gewählten Ausgangsmaterial weitere Alkyl- und Alkenylreste anderer Kettenlängen in dem Substanzgemisch untergeordnet vorliegen können.

Die quaternären Zuckerderivate mit der Formel (II) schließen explizit auch Oligosaccharidverbindungen ein, bei denen die Anzahl der Monosaccharideinheiten n bevorzugt 1 < n < 100 ist. R₁ stellt bevorzugt ein Wasserstoffatom und R₂ bevorzugt eine Alkylgruppe mit C₈-C₂₀ und besonders bevorzugt C₁₈ Kohlenstoffatomen dar.

Das Anion X⁻ in der oben angegebenen Formel (II) ist vorzugsweise ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, lodid, Saccharinat, Tosylat oder Methosulfat, ganz besonders bevorzugt Chlorid.

### c) Positiv geladene Phospholipide, bestehend aus Diester- und Triesterphosphatiden mit der Formel (III),

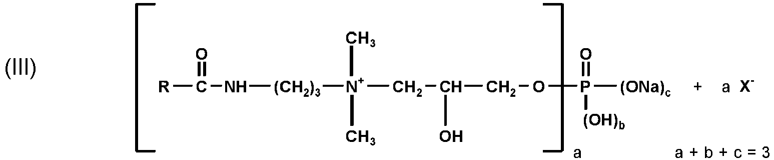

Die positiv geladenen Phospholipide und deren Salze mit der Formel (III) weisen einen unverzweigten oder verzweigten Alkylrest und/oder Alkenylrest R mit C₇-C₂₅ und besonders bevorzugt mit C₁₃-C₂₁ Kohlenstoffatomen auf. Ganz besonders bevorzugt weist der Alkylrest R C₁₃, C₁₇ und/oder C₂₁ Kohlenstoffatome auf. Im Falle eines Alkenylrestes weist R besonders bevorzugt Ketten mit C₁₇ Kohlenstoffatomen auf. Dabei liegen im Alkenylrest bevorzugt ein, zwei oder drei Doppelbindungen vor.

Zur Herstellung dieser Stoffe werden vornehmlich natürliche Öle, bevorzugt Kokosöl, Palmöl/Palmkernöl, Sonnenblumenöl, Distelöl, Olivenöl, Traubenkernöl, Borretschöl und Rizinusöl etc. herangezogen, so dass neben den primär genannten unverzweigten oder verzweigten Alkyl- und/oder Alkenylresten R entsprechend dem gewählten Ausgangsmaterial weitere Alkenyl- und Alkylreste anderer Kettenlängen in dem Substanzgemisch untergeordnet vorliegen können. Im Falle von Rizinusöl weist der C₁₇ Alkenylrest eine funktionelle Hydroxylgruppe auf.

Das Anion X⁻ in der oben angegebenen Formel ist vorzugsweise ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, lodid, Saccharinat, Tosylat oder Methosulfat, ganz besonders bevorzugt Chlorid.

### d) Positiv geladene N-(3-Alkylamido)propyl-N,N-dimethyl-N-(2,3-dihydroxypropyl)-ammoniumsalze oder N-(3-Alkenylamido)propyl-N,N-dimethyl-N-(2,3-dihydroxy-propyl)-ammoniumsalze mit der Formel (IV)

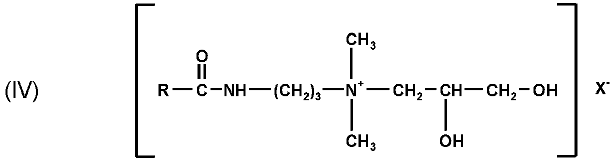

oder N-(3-Ricinylamido)propyl-N,N-dimethyl-N-(2,3-dihydroxypropyl)-ammoniumsalze.

Die positiv geladenen N-(3-Alkylamido)propyl-N,N-dimethyl-N-(2,3-dihydroxypropyl)-ammoniumsalze der Formel (IV) weisen bevorzugt einen unverzweigten oder verzweigten Alkylrest R mit C₇-C₂₅ und besonders bevorzugt mit C₁₃-C₂₁ Kohlenstoffatomen auf. Ganz besonders bevorzugt weist der Alkylrest C₁₃, C₁₇ und/oder C₂₁ Kohlenstoffatome auf. Bei den N-(3-Alkylamido)propyl-N,N-Dimethyl-N-(2,3-Dihydroxypropyl)-ammoniumsalzen mit der Formel (IV) kann es sich um einheitliche Substanzen handeln, in der Regel werden jedoch zur Herstellung dieser Stoffe vornehmlich natürliche Öle, bevorzugt Kokosöl und Palmöl/Palmkernöl, gegebenenfalls aber auch Talg herangezogen, so dass neben den primär genannten unverzweigten oder verzweigten Alkylresten R entsprechend dem gewählten Ausgangsmaterial weitere Alkyl- und Alkenylreste anderer Kettenlängen in dem Substanzgemisch untergeordnet vorliegen können.

Das Anion X⁻ in der oben angegebenen Formel ist vorzugsweise ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, lodid, Saccharinat, Tosylat oder Methosulfat, ganz besonders bevorzugt Chlorid.

Die N-(3-Alkenylamido)propyl-N,N-Dimethyl-N-(2,3-Dihydroxypropyl)-ammoniumsalze mit der Formel (IV) weisen bevorzugt einen unverzweigten oder verzweigten Alkenylrest R mit C₁₃-C₂₃ und besonders bevorzugt mit C₁₇ Kohlenstoffatomen auf. Dabei liegen im Alkenylrest bevorzugt ein, zwei oder drei Doppelbindungen vor.

Bei den N-(3-Alkenylamido)propyl-N,N-Dimethyl-N-(2,3-Dihydroxypropyl)-ammoniumsalzen mit der Formel (IV) kann es sich um einheitliche Substanzen handeln, in der Regel werden jedoch zur Herstellung dieser Stoffe vornehmlich natürliche Öle, bevorzugt Sonnenblumenöl, Distelöl, Olivenöl, Traubenkernöl, Borretschöl und Rizinusöl etc. herangezogen, so dass neben den primär genannten unverzweigten oder verzweigten Alkenylresten R entsprechend dem gewählten Ausgangsmaterial weitere Alkenyl- und Alkylreste anderer Kettenlängen in dem Substanzgemisch untergeordnet vorliegen können.

Das Anion X⁻ in der oben angegebenen Formel ist vorzugsweise ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, lodid, Saccharinat, Tosylat oder Methosulfat, ganz besonders bevorzugt Chlorid.

### e) Positiv geladene quarternäre N,N,N-Trimethyl-N-(2-hydroxy-3-"R-Ether"-propyl)-ammoniumsalze mit der Formel (V)

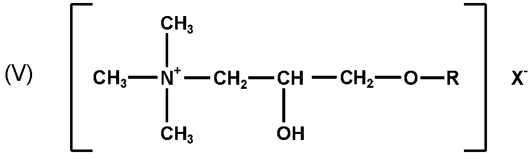

Die quarternären N,N,N-Trimethyl-N-(2-Hydroxy-3-*"R-Ether"*-propyl)-ammoniumsalze mit der Formel (V) enthalten als Rest R Hyaluronsäure, Xanthan-Gummi oder Trehalose. Das Anion X⁻ in der oben angegebenen Formel ist vorzugsweise ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, lodid, Saccharinat, Tosylat oder Methosulfat.

Wahlweise können die Vesikel zusätzlich zu den oben aufgeführten Ladungsgebern noch einen oder mehrere der folgenden weiteren Ladungsgeber aufweisen:

### f) Positiv geladene quarternäre C₁₂-C₂₂-Alkyl-trimethylammoniumsalze (bzw. Fettsäure-Trimoniumsalze) mit der Formel (VI)

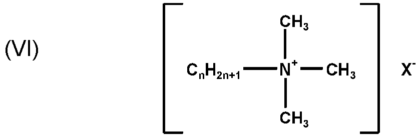

Die quarternären Alkyl-Trimethylammoniumsalze (bzw. Fettsäure-Trimoniumsalze) mit der Formel (VI) weisen bevorzugt einen Alkylrest mit C₁₂-C₂₂ Kohlenstoffatomen auf. Vorzugsweise ist n in der oben angegebenen Alkyl-Trimethylammoniumsalzformel gleich 22. X⁻ ist in der oben angegebenen Formel (VI) ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, lodid, Saccharinat, Tosylat oder Methosulfat.

Es konnte gezeigt werden, dass die erfindungsgemäßen Ladungsgeber den Vorteil bieten, dass große Mengen lipophiler UV-Filtersubstanz stabil in ein nano-skaliges, neuartiges Trägersystem eingebracht werden können und so die Wirkung des UV-Schutzmittels optimieren.

Ein weiterer Vorteil besteht darin, dass durch die Ladungsgeber der vorliegenden Erfindung die Adhäsion der Vesikel an Haut und Haar und damit die Verweildauer der UV-Filtersubstanz signifikant gesteigert werden kann. Die erfindungsgemäße Zubereitung zeigt in dieser Hinsicht nachweislich eine gute Filmbildung auf Haut und Haar, sowie eine mehrere Waschgänge überdauernde effektive Adhäsion. Sowohl die nach mehreren Waschgängen auf Haut und Haar verbleibende Menge an UV-Filtersubstanz als auch deren Schutzwirkung konnten durch die erfindungsgemäße Zubereitung gegenüber freien UV-Filtersubstanzen in erheblichem Maße gesteigert werden.

Zum anderen wird durch die Verkapselung der UV-Filtersubstanz eine Einarbeitung in hoher und wirksamer Konzentration in hydrophile kosmetische und/oder pharmazeutische Formulierungen ermöglicht. Auch die Kombination mehrerer öl-löslicher UV-Filtersubstanzen mit synergistischem Wirkspektrum in einem Trägersystem ist durch ein UV-Schutzmittel mit den erfindungsgemäßen Eigenschaften möglich. Auf diese Weise kann ein äußerst effektiver Breitbandschutz über den UVA- und UVB-Bereich erreicht werden.

Der Begriff "Verkapselung" umfasst im Zusammenhang mit der vorliegenden Erfindung die prinzipielle Einbettung von UV-Filtersubstanzen zwischen den Polysaccharidmolekülen und den von den Polysaccharidmolekülen ausgebildeten Nanostrukturen sowie den Einschluss der UV-Filtersubstanzen im Vesikelinneren.

Die erfindungsgemäßen Lipidvesikel haben ein Zetapotential im Bereich von 1 bis 150 mV. Der Begriff "Zetapotential" beschreibt das elektrische Potential einer Abscherschicht eines bewegten Partikels in einer Suspension. Die Messung des Zetapotentials kann dadurch erfolgen, dass man Partikel durch ein angelegtes elektrisches Feld bewegt. Aus der resultierenden Geschwindigkeit der Partikel lässt sich dann das Zetapotential berechnen. Bevorzugte Lipidvesikel weisen ein Zetapotential von 30 bis 100 mV auf. Bei besonders bevorzugten Lipidvesikeln beträgt das Zetapotential 40 bis 60 mV.

Das Zetapotential wird im Zusammenhang mit der vorliegenden Erfindung mittels Laser-Doppler-Elektrophorese bestimmt. Dabei erfolgen die Messungen in stark verdünnten wässrigen Kochsalzlösungen, wobei die zu vermessenden Proben üblicherweise in Konzentrationen von 0,01-0,1 Gew.-% in 1 mM Natriumchloridlösung vorliegen. Die pH-Werte der Probenlösungen liegen im pH-Wert-Spezifikationsbereich der jeweiligen zu vermessenden Produkte (erfindungsgemäße UV-Schutzmittel und Formulierungen), wobei im Regelfall produktspezifisch pH-Werte im Bereich von pH 5,5 bis maximal pH 7,5 getroffen werden.

Die verbesserte Adhäsion des UV-Schutzmittels basiert u.a. auf der positiven Ladung der Vesikeloberfläche. Die positive Ladung der Vesikeloberfläche führt zu einer verbesserten Anhaftung der Vesikel an der Oberfläche von Hautzellen. Darüber hinaus führt die positive Ladung der Vesikel auch zu einer starken Anhaftung an der Haaroberfläche. Es hat sich außerdem gezeigt, dass diese verbesserte Adhäsion auch mit der erfindungsgemäß nano-skaligen Teilchengröße der Trägervesikel zusammenhängt, wobei all diese Feststellungen in Bezug auf die vorliegende Erfindung jedoch nicht bindend sein sollen und den Umfang der Erfindung daher auch nicht beschränken sollen.

Die Teilchengröße der erfindungsgemäßen Lipidvesikel ist in dem Bereich von 10 bis 1000 nm. Bei bestimmten Ausführungsformen ist die Teilchengröße 100 bis 400 nm. Bei anderen Ausführungsformen ist sie 100 bis 350 nm. Bei bevorzugten Ausführungsformen ist die Teilchengröße 100 bis 250 nm.

Der Begriff "Teilchengröße" bedeutet im Zusammenhang mit der vorliegenden Erfindung die mittlere Teilchengröße. Die mittlere Teilchengröße ist mittels Photonenkorrelationsspektroskopie bestimmbar. Die Photonenkorrelationsspektroskopie - auch als dynamische Lichtstreuung bezeichnet - ist ein optisches Messverfahren zur Bestimmung der Größenverteilung von Vesikeln und Partikeln in Flüssigkeiten. Die Methode nutzt die Streuung von Laserlicht durch die Vesikel aus.

Durch die erfindungsgemäße Kombination von positiver Ladung der Vesikeloberfläche und nanoskaliger Teilchengröße der aus hydrophobierten Polysacchariden gebildeten Trägervesikel wird sowohl bei Haut- als auch bei Haaranwendungen erreicht, dass in den Vesikeln enthaltene UV-Filtersubstanz/en dauerhafter auf den zu schützenden Oberflächen verbleiben. Allerdings penetrieren diese Vesikel die Haut bzw. die Haare nicht. Das heisst, diese Vesikel dringen nicht in die Haut bzw. die Haare ein, was mit Hilfe von mit Fluoreszenzfarbstoff beladenem Trägersystem durch Fluoreszenzmikroskopie nachgewiesen werden kann.

Vorzugweise werden bei der vorliegenden Erfindung einer oder mehrere der vorgenannten positiv geladenen Ladungsgeber so eingesetzt, dass der Ladungsgeberanteil in den Vesikeln bezogen auf das Gesamtgewicht der Vesikel insgesamt in dem Bereich von 0,01 bis 10 Gew.-% liegt. Bei bestimmten Ausführungsformen liegt der Ladungsgeberanteil in dem Bereich von 0,01 bis 2,0 Gew.-%.

Die in Gewichtsprozenten angegebenen Anteile beziehen sich prinzipiell auf die vollständige Formulierung des UV-Schutzmittels gemäß Rezepturvorgaben, inklusive des zur Vesikelbildung erforderlichen Wasseranteils, wenn nicht etwas anderes angegeben ist.

Der angegebene Anteil umfasst außerdem alle eindeutig unter die Definition der jeweiligen Substanzgruppe fallenden Substanzen. Demnach umfasst der angegebene Ladungsgeberanteil bei den Ausführungsformen mit einem Ladungsgeber den Anteil dieses einen Ladungsgebers und bei Ausführungsformen mit mehreren Ladungsgebern die Summe aller Ladungsgeber. Die gilt im Übrigen in gleicher Weise für im Zusammenhang mit dieser Erfindung angegebene Anteile an hydrophobiertem Polysaccharid, Filtersubstanz und Hilfsstoff oder anderen Substanz- bzw. Stoffanteilen.

Das Polysaccharidgrundgerüst der hydrophobierten Polysaccharide, die bei dem UV-Schutzmittel für die Bildung der Vesikel verwendet werden können, kann unter allen kosmetisch oder pharmazeutisch verträglichen Polysacchariden ausgewählt werden, die in der Lage sind, Vesikel zu bilden. Vorzugsweise handelt es sich um wasserlösliche Polysaccharide und/oder um deren Ether mit kurzkettigen Alkoholen (C₁ bis C₄), wobei die wasserlöslichen Polysaccharide linear, verzweigt, kammartig und/oder sternförmig sein können. Besonders bevorzugt sind lineare wasserlösliche Polysaccharide. Es kommen auch Copolymere oder Block-Copolymere unterschiedlicher Monosaccharid-Einheiten und/oder auf unterschiedliche Weise miteinander verknüpfter Monosaccharid-Einheiten in Betracht.

Die hydrophobierten Polysaccharide, aus denen die Vesikel aufgebaut sind, weisen vorzugsweise ein Polysaccharid-Grundgerüst aus Polyglucose oder Polyfructose auf. Bevorzugte hydrophobierte Polysaccharide mit einem Polysaccharid-Grundgerüst aus einer Polyglucose sind Cellulose, Methylcellulose, Hydroxyethylcellulose, Amylose, Amylopektin und Dextrin. Ein bevorzugtes hydrophobiertes Polysaccharid mit einem Polysaccharid-Grundgerüst aus Polyfructose ist Inulin.

Das hydrophobierte Polysaccharid umfasst bei der vorliegenden Erfindung vorzugsweise im Mittel von 5 bis 1000 Monosaccharid-Einheiten. Noch bevorzugter sind von 10 bis 500 Monosaccharid-Einheiten. Insbesondere bevorzugt sind von 20 bis 100 Monosaccharid-Einheiten.

Bei speziellen Ausführungsformen können auch Gemische der oben genannten Polysaccharide eingesetzt werden mit der Maßgabe, dass diese Gemische in der Lage sind, Vesikel zu bilden.

Der Anteil der Polysaccharide in den Vesikeln liegt bezogen auf das Gesamtgewicht der Vesikel in dem Bereich von 1 bis 85 Gew.-%. Vorzugsweise beträgt der Polysaccharidanteil bezogen auf das Gesamtgewicht der Vesikel 5 bis 25 Gew.-%. Besonders bevorzugt beträgt der Polysaccharidanteil 8 bis 15 Gew.-%.

Bei bevorzugten Ausführungsformen sind die hydrophobierten Polysaccharide, aus denen die Vesikel aufgebaut sind, dadurch hydrophobiert, dass sie ein Polysaccharid-Grundgerüst mit C₃₋₂₂-Alkylgruppen, die über Alkyl-Etherbindungen oder über Alkyl-Urethanbindungen an die Hydroxygruppen des Polysaccharids gebunden sind, oder die über einen Linker (z.B. Polyether-Linker, Polyethylenglykol-Linker) an das Polysaccharid-Grundgerüst gebunden sind, aufweisen.

Das Molekulargewicht des hydrophob modifizierten Polysaccharids liegt bei bevorzugten Ausführungsformen in dem Bereich von 5000 bis 500.000 g/mol. Bevorzugt ist hierbei der Bereich von 5000 bis 100.000 g/mol.

Bei bevorzugten hydrophob modifizierten Polysacchariden beträgt der Quotient aus Anzahl an hydrophob modifizierenden Gruppen und modifizierbaren Gruppen (Modifizierungsgrad) von 0,01 bis 0,9. Bei besonders bevorzugten Ausführungsformen beträgt der Modifizierungsgrad von 0,03 bis 0,15.

Die hydrophoben Gruppen und auch das Polysaccharid-Rückgrat können bei bestimmten Ausführungsformen durch beispielsweise Halogen-, Hydroxy-, Alkoxy-, Amino-, Alkylamino-, Aryl-, Arylalkyl-, Carboxy-, Carboxyester- und cycloaliphatische Reste ein- oder mehrfach substituiert sein. Bevorzugt handelt es sich bei den hydrophob modifizierten Polysacchariden jedoch um nichtionische Verbindungen.

Erfindungsgemäß besonders bevorzugt eingesetzte hydrophob modifizierte Polysaccharide sind Inulinlaurylcarbamate, Cetylhydroxyethylcellulose, Hydroxy-C3-6-alkyl-modifizierte Cellulose, insbesondere Hydroxypropylcellulose und Hydroxypropylmethylcellulose.

Als UV-Filtersubstanz kommen bei der vorliegenden Erfindung uneingeschränkt alle chemisch oder physikalisch UV-filternd wirkenden Substanzen in Betracht, vorausgesetzt, sie sind lipophil.

Eine UV-Filtersubstanz ist im Sinne der vorliegenden Erfindung dann lipophil, wenn Sie bei 25°C einen n-Oktanol-Wasser-Verteilungskoeffizient *K*_{ow} aufweist, der größer als 1 ist.

Die chemischen Filtersubstanzen absorbieren energiereiche Strahlung und geben sie als energieärmere, langwelligere Strahlung oder Wärme wieder ab. Die physikalischen Filtersubstanzen streuen und reflektieren das Licht hauptsächlich.

Bevorzugte Beispiele für UV-Filtersubstanzen, die bei der vorliegenden Erfindung zur Anwendung kommen können, sind ohne Beschränkung hierauf 3-Benzylidencampher, 4-Methylbenzylidencampher, Benzophenon-1, Benzophenon-2, Benzophenon-3, Benzophenon-4, Benzophenon-5, Benzophenon-6, Benzophenon-9, Benzylidencamphersulfonsäure, Bis-Ethylhexyloxyphenolmethoxyphenyltriazin, Butylmethoxydibenzoylmethan, Campherbenzalkoniummethosulfat, Diethylaminohydroxybenzoylhexylbenzoat, Diethylhexylbutamidotriazon, Dinatriumphenyldibenzimidazoltetrasulfonat, Drometrizoltrisiloxan, Ethylhexyldimethylpaba, Ethylhexylmethoxycinnamat, Ethylhexylsalicylat, Ethylhexyltriazon, Homosalat, Isoamyl-p-methoxycinnamat, Methylen-bis-benzotriazolyltetramethylbutylphenol, Octocrylen, PEG-25-Paba, Phenylbenzimidazolsulfonsäure, Polyacrylamidomethylbenzylidencampher, Polysilicon-15, Kalium-phenylbenzimidazolsulfonat, Natriummangoseedat, Natriumphenylbenzimidazolsulfonat, Tea-Phenylbenzimidazolsulfonat, Terephthalylidendicamphersulfonsäure, Ferulasäure, Cinoxat, Diisopropylmethylcinnamat, 4-(2-Beta-Glucopyranosiloxy)propoxy-2-hydroxybenzophenon, Glycerylethylhexanoatdimethoxycinnamat, Isopentyltrimethoxycinnamattrisiloxan.

Vorzugsweise beträgt der Anteil an UV-Filtersubstanz bei der vorliegenden Erfindung 1 bis 65 Gew.-% bezogen auf das Gesamtgewicht der vollständigen Formulierung des UV-Schutzmittels gemäß Rezepturvorgaben, inklusive des zur Vesikelbildung erforderlichen Wasseranteils.

Da die einzelnen Filtersubstanzen in der Regel keinen Schutz über das gesamte UV-Spektrum hinweg bieten, werden bei bevorzugten Ausführungsformen der Erfindung mehrere Filtersubstanzen kombiniert, um einen möglichst breiten UV-Schutz zu erreichen.

Erfindungsgemäß wird das UV-Schutzmittel in kosmetischen und/oder pharmazeutischen Formulierungen verwendet, wobei pharmazeutische Formulierungen solche sind, die unter das Arzneimittelrecht fallen.

Die Formulierungen können alle Hilfs- und Zusatzstoffe, die üblicherweise bei kosmetischen oder pharmazeutischen Präparaten Anwendung finden, enthalten. Insbesondere fallen unter den Begriff "Hilfsstoff' im Zusammenhang mit der vorliegenden Erfindung solche Zusatzstoffe, die auf die physikalischen Eigenschaften der Vesikel und deren Stabilität einwirken und/oder der Konservierung der UV-Schutzmittel dienen. Beispiele für solche Hilfsstoffe sind Öle, Alkohole, Polyole, Antioxidantien, Gelbildner, Puffer, Konservierungsmittel, Bakterizide und Keimhemmer, Konsistenzgeber, Verdicker und Komplexbildner.

Vorzugsweise ist das UV-Schutzmittel gemäß der vorliegenden Erfindung dadurch gekennzeichnet, dass der Hilfsstoffanteil bezogen auf das Gesamtgewicht der vollständigen Formulierung des UV-Schutzmittels gemäß Rezepturvorgaben, inklusive des zur Vesikelbildung erforderlichen Wasseranteils in dem Bereich von 0,01 bis 10 Gew.-% liegt.

Die Formulierung mit dem erfindungsgemäßen UV-Schutzmittel umfasst zusätzlich ein die Haut oder die Haare penetrierendes liposomales Trägersystem, in dem wenigstens ein antioxidativer Wirkstoff verkapselt ist. Der antioxidative Wirkstoff verhindert oder hemmt die chemisch oder photochemisch induzierte Oxidation empfindlicher Moleküle, die Rezepturbestandteil aber insbesondere auch Bestandteil eines hautphysiologischen oder haarphysiologischen Systems sein können, indem er als Radikalfänger Radikalkettenreaktionen terminiert oder als reduzierende Substanz mit sehr geringem eigenem Redoxpotential leichter und schneller oxidiert wird als die zu schützenden Moleküle bzw. Systeme oder aber in synergistischer Weise die Wirkung anderer Antioxidantien unterstützt, in dem er Protagonisten der oxidativen Prozesse z.B. durch Chelatisierung inaktiviert.

Demnach wird bei der Herstellung der Formulierung mit dem erfindungsgemäßen UV-Schutzmittel bei einer bestimmten Ausführungsform zusätzlich ein die Haut oder die Haare penetrierendes liposomales Trägersystem, in dem wenigstens ein antioxidativer Wirkstoff verkapselt ist, verwendet.

Vorzugsweise besteht das vorgenannte liposomale Trägersystem aus Vesikeln, die aus Lipiden aufgebaut sind. Besonders bevorzugt weisen die Vesikel eine Teilchengröße von 10 bis 1000 nm auf.

Die Lipide, aus denen die liposomalen Vesikel aufgebaut sind, sind vorzugsweise unter Ceramiden, Phospholipiden, Glycosphingolipiden und/oder Di-Acylglycosiden ausgewählt.

Vorzugsweise beträgt der Anteil der Lipide, aus denen die liposomalen Vesikel aufgebaut sind, bezogen auf die gesamte Formulierung 1 bis 20 Gew.-%.

Der wenigstens eine antioxidative Wirkstoff, der in den liposomalen Vesikeln verkapselt ist, ist vorzugsweise ausgewählt unter lipophilen und hydrophilen antioxidativen Substanzen, die aus natürlichen Quellen isoliert oder chemisch oder biotechnologisch hergestellt wurden, oder Kombinationen davon. Besonders bevorzugt ist der wenigstens eine antioxidative Wirkstoff - ohne Beschränkung hierauf - ausgewählt aus der Gruppe der Vitamine, bevorzugt Ascorbinsäure (Vitamin C), deren Salze, deren 2-O- und/oder 3-O- Etherverbindungen, ganz bevorzugt 3-O-Ethyl Ascorbinsäure, sowie Mono-, Di- und Tetraester der Ascorbinsäure mit Palmitinsäure, Stearinsäure, Isostearinsäure, Linolsäure, Schwefelsäure und Phosphorsäure sowie deren Alkali- und Erdalkalisalzen und Ascorbyl-2-O-Glucosid, Tocopherol (Vitamin E), dessen Ester mit Essigsäure, Ferulasäure, Linolsäure, Ölsäure, Nicotinsäure, Retinsäuren, Bernsteinsäure, Maleinsäure und Phosphorsäure sowie deren Alkali- und Erdalkalisalzen, Tocotrienol und Tocopheryl-6-O-D-glucopyranosid.

Alternativ kann der wenigstens eine antioxidative Wirkstoff - ohne Beschränkung hierauf - ausgewählt sein aus der Gruppe der kosmetisch oder pharmazeutisch verträglichen Phenolverbindungen mit einer oder mehreren Hydroxylgruppen, bevorzugt Tert-butyl-4-methoxyphenol (BHA), 2,6-Di-tert-butyl-p-kresol (BHT) und Derivaten des Resorcinols, z.B. 4-Butylresorcinol, 4-(1-Phenylethyl)resorcinol, etc. und kosmetisch oder pharmazeutisch verträglichen Derivaten des Hydrochinons, z.B. Ubichinol, dessen oxidierte chinoide Form 6-all-*trans*-Decaprenyl-2,3-dimethoxy-5-methyl-1,4-benzochinon ("Coenzym Q10") und seine Alkalisalze, 6-(10-Hydroxydecyl)-2,3-dimethoxy-5-methyl-1,4-benzochinon ("Idebenon") und dessen Linolsäureester sowie Hydroxylgruppen-haltige Benzoesäurederivate (z.B. Vanillinsäure, Gallussäure, Protocatechusäure etc.) und Zimtsäurederivate (z.B. Kaffeesäure, p-Cumarsäure, etc.) sowie deren Ester mit C₂-C₁₈ Alkoholen bzw. Fettalkoholen.

Außerdem kann der wenigstens eine antioxidative Wirkstoff - ohne Beschränkung hierauf - auch ausgewählt sein aus der Gruppe der Hydroxylgruppen-haltigen Stilbenderivate (z.B. Resveratrol) sowie Ellagsäure und Rosmarinsäure.

Besonders bevorzugt ist der wenigstens eine antioxidative Wirkstoff - ohne Beschränkung hierauf - ausgewählt aus der Gruppe der Polyphenolverbindungen, wobei hierbei die Untergruppen ein chemisches Strukturgerüst der Chalkone, Flavone (z.B. Luteolin), Flavonole (z.B. Quercetin, Rutin etc.), Flavanole (Catechin, Gallocatechin, Epicatechin, Epigallocatechingallat, dimere und trimere Catechine OPC, Tannine, etc.), Flavanone (z.B. Hesperetin), Flavanonole, Isoflavone (z.B. Genistein, Licoricidin etc.), Anthocyane und Aurone aufweisen sowie vornehmlich die Glykoside der o.g. Verbindungsgruppen.

Da es sich bei den oben aufgeführten Polyphenolverbindungen um sekundäre Pflanzenstoffe handelt, ist der wenigstens eine antioxidative Wirkstoff Wirkstoff - ohne Beschränkung hierauf - besonders bevorzugt ein wässriger, alkoholischer, hydroglykolischer, öliger und/oder CO₂-Extrakt polyphenolreicher Pflanzen- und Pflanzenteile, wobei in Abhängigkeit der Pflanzenart Wurzeln, Stängel, Blätter, Blüten, Früchte, Fruchtschalen und/oder Samen als Extraktionsgrundlage dienen können. Die Extrakte können flüssig, aufkonzentriert und/oder nach Sprüh- oder Gefriertrocknung auch als Feststoff vorliegen. Naturgemäß enthalten diese Extrakte dann in der Regel keine isolierte einheitliche Substanz sondern vielmehr Substanzgemische der oben aufgeführten Verbindungen in variabler Zusammensetzung.

Besonders bevorzugt sind die polyphenolreichen Pflanzenextrakte ausgewählt unter Rosmarinextrakten, Ingwerextrakten, Thymianextrakten, Salbeiextrakten, Tee-, vornehmlich Grünteeextrakten, Traubenkern- und Traubenschalenextrakten, Apfelbeerenextrakten, Granatapfelextrakten, Rooibosextrakten, Gallapfelextrakten, Hopfenextrakten, Ginkgoextrakten, Melissenextrakten, etc.. Bei weiteren alternativen Ausführungsformen der Erfindung ist der wenigstens eine antioxidative Wirkstoff ausgewählt ist aus der Gruppe der antioxidativ wirksamen Enzyme, bevorzugt Superoxiddismutase (SOD), Glutathionperoxidase (GPX) und Katalase. Bei noch einer weiteren alternativen Ausführungsform der Erfindung ist der wenigstens eine antioxidative Wirkstoff ausgewählt ist aus der Gruppe der Sulfide und Sulfite, bevorzugt Glutathion und Alkali- und Erdalkalisulfite und -disulfite und Alkalibisulfite.

Der Anteil des wenigstens einen antioxidativen Wirkstoffs bezogen auf die gesamte Formulierung beträgt vorzugsweise 0,01 - 20 Gew.-%.

Bei den Ausführungsformen der Erfindung mit zusätzlichem liposomalen Trägersystem weist die Formulierung vorzugsweise zusätzlich einen oder mehrere Hilfsstoffe auf, die bevorzugt ausgewählt sind unter: Chelatbildner wie Ethylendiamintetraessigsäure (EDTA) und/oder ihre Salze, Nitrilotriessigsäure (NTA) und/oder ihre Salze, kosmetisch oder pharmazeutisch verträgliche Phosphate und/oder Phosphonate, Salze und Ester der Oxalsäure und/oder Weinsäure sowie besonders bevorzugt N,N- Bis(carboxymethyl)glutaminsäure und/oder ihre Salze, etc. eingesetzt, da durch sie die Wirkung anderer Antioxidantien unterstützt wird, in dem Protagonisten der oxidativen Prozesse durch Chelatisierung inaktiviert werden.

Bevorzugt wird das erfindungsgemäße UV-Schutzmittel für kosmetische und/oder pharmazeutische Formulierungen verwendet, die zur topischen Applikation geeignet sind. Das erfindungsgemäße UV-Schutzmittel kann hierfür in allen für die topische Applikation geeigneten Formulierungen vorliegen, beispielsweise in Form eines Gels, einer Creme, einer Salbe, eines Sprays, einer Lotion, einer wässrigen oder wässrig-alkoholischen Zubereitung. Dazu kann das erfindungsgemäße UV-Schutzmittel in eine Trägermatrix eingearbeitet werden. Bei der Trägermatrix kann es sich um Gelformulierungen, Cremeformulierungen, Lotionen, Maskenanwendungen, Tinten und Wachsstiftformulierungen etc. handeln.

Für Anwendungen im Hautbereich wird das erfindungsgemäße UV-Schutzmittel vorzugsweise in einer Lotion, einer Creme, einer Salbe, einem Gel, einem wässrigen Fluid, einem Gesichtswasser, einem Sonnenprodukt oder einer Maske angewendet.

Für Anwendungen im Haarbereich wird das erfindungsgemäße UV-Schutzmittel vorzugsweise in einem Shampoo (vorzugsweise mit milden Tensiden), einer Spülung, einem Haargel, einem Conditioner, einem Hair Tonic, einem Haar-Styling-Produkt oder einem Haar- Pflege-Produkt angewendet.

Selbstverständlich handelt es sich bei allen Komponenten des erfindungsgemäßen UV-Schutzmittels und von dessen Formulierungen um pharmazeutisch, kosmetisch oder dermatologisch verträgliche Stoffe. Ein Stoff ist im Sinne dieser Erfindung pharmazeutisch, kosmetisch oder dermatologisch verträglich, wenn er nicht toxisch ist und bei der Mehrzahl der potentiellen Anwender topisch anwendbar ist, ohne dass es bei dem Anwender spontan oder nach einer Weile zu einer ungewünschten physiologischen Reaktion, wie z.B. einer Rötung oder der Ausbildung eines Juckreizes kommt.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische, kosmetische, pharmakologische oder dermatologische Gegebenheiten derartige Kombinationen unmöglich oder sinnlos machen. Auf die umfassende, explizite Darstellung sämtlicher denkbaren Merkmalskombinationen wird hier nur der Kürze und der Lesbarkeit der Beschreibung wegen verzichtet.

## Patentansprüche

1. Kosmetische oder pharmazeutische Formulierung, die ein UV-Schutzmittel und zusätzlich einen antioxidativen Wirkstoff enthält, **dadurch gekennzeichnet, dass** der wenigstens eine antioxidative Wirkstoff in einem die Haut oder die Haare penetrierenden liposomalen Trägersystem verkapselt ist und das UV-Schutzmittel für die topische Applikation auf die Haut oder die Haare wenigstens eine UV-Filtersubstanz umfasst, die in einem vesikulären, die Haut oder die Haare nicht penetrierenden Trägersystem verkapselt ist, wobei die wenigstens eine UV-Filtersubstanz bei 25°C einen *n*-Oktanol-Wasser-Verteilungskoeffizient *K_{ow}* aufweist, der größer als 1 ist und das vesikuläre Trägersystem aus Vesikeln besteht, die aus hydrophobierten Polysacchariden aufgebaut sind und eine Teilchengröße von 10 bis 1000 nm sowie eine positive Oberflächenladung mit einem Zetapotential in dem Bereich von 1 bis 150 mV aufweisen, wobei die hydrophobierten Polysaccharide, aus denen die Vesikel aufgebaut sind, dadurch hydrophobiert sind, dass die C₃₋₂₂-Alkylgruppen über die Alkyl-Etherbindungen oder über Alkyl-Urethanbindungen an die Hydroxygruppen des Polysaccharids gebunden sind oder über einen Linker an das Polysaccharid-Grundgerüst gebunden sind, und wobei die positive Oberflächenladung dadurch bewirkt wird, dass die Vesikel zusätzlich zu den Polysacchariden, aus denen die Vesikel aufgebaut sind, positiv geladene Moleküle als Ladungsgeber aufweisen, wobei diese Ladungsgeber ausgewählt sind unter:
a) N-Stearyl-N,N-Dimethyl-N-(2-Hydroxy-3-panthenyl)propyl-ammoniumsalzen mit der Formel (I), 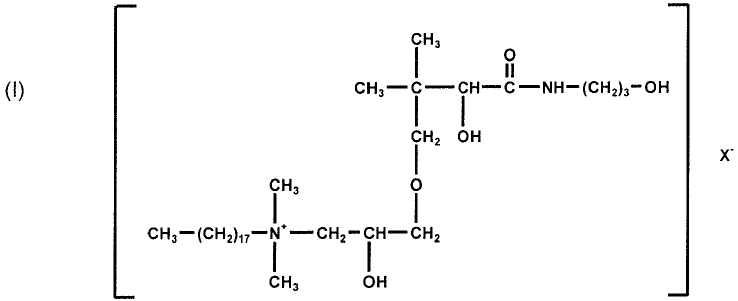
b) positiv geladenen quaternären Zuckerderivaten mit der Formel (II) 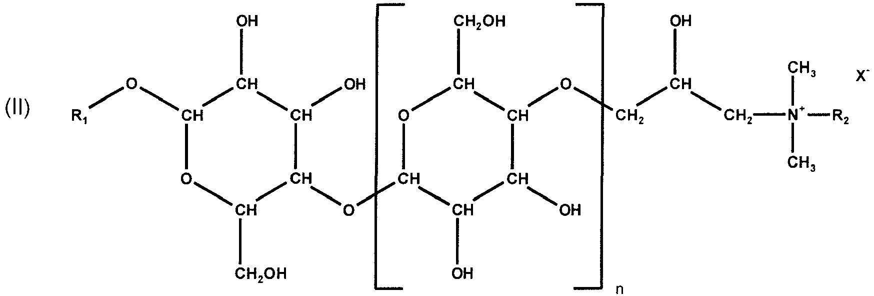 worin R₁ und R₂ Alkylreste sind,
c) positiv geladenen Phospholipiden, ausgewählt unter Diester- und Triesterphosphatiden mit der Formel (III), 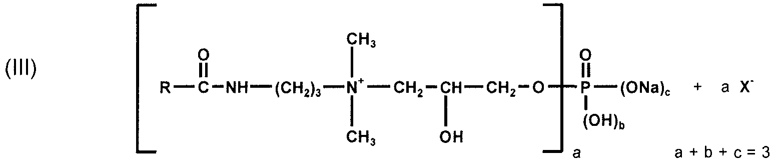 wobei der Rest R ein unverzweigter oder verzweigter Alkylrest und/oder Alkenylrest mit C₇-C₂₅ Kohlenstoffatomen ist,
d) positiv geladenen N-(3-Alkylamido)propyl-N,N-dimethyl-N-(2,3-dihydroxypropyl)-ammoniumsalzen und N-(3-Alkenylamido)propyl-N,N-dimethyl-N-(2,3-dihydroxypropyl)-ammoniumsalzen mit der Formel (IV) 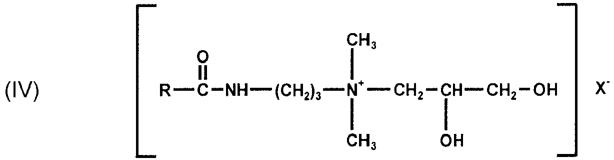 sowie N-(3-Ricinylamido)propyl-N,N-dimethyl-N-(2,3-dihydroxypropyl)-ammoniumsalzen,
e) positiv geladenen quarternären N,N,N-Trimethyl-N-(2-hydroxy-3-*"R*-*Ether"*-propyl)-ammoniumsalzen mit der Formel (V) 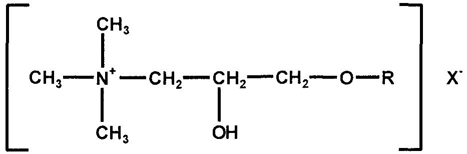 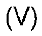
worin der Rest R Hyaluronsäure, Xanthan-Gummi oder Trehalose repräsentiert,
oder Kombinationen davon,
und wobei die Vesikel zur Ausbildung der positiven Oberflächenladung wahlweise zusätzlich zu den oben angegebenen Ladungsgebern noch
f) ein oder mehrere positiv geladene quarternäre C₁₂-C₂₂-Alkyl-Trimethylammoniumsalze (bzw. Fettsäure-Trimoniumsalze) mit der Formel 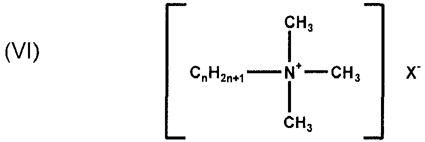 aufweisen,
wobei X⁻ in den oben angegebenen Formeln (I) bis (VI) ein kosmetisch oder pharmazeutisch verträgliches organisches oder anorganisches Anion ist.

2. Formulierung gemäß einem der vorangehenden Ansprüche, **dadurch gekennzeichnet, dass** der UV-Filtersubstanzanteil in den Vesikeln bezogen auf das Gesamtgewicht des UV-Schutzmittels in dem Bereich von 1 bis 65 Gew.-% liegt.

3. Formulierung gemäß einem der vorangehenden Ansprüche, **dadurch gekennzeichnet, dass** der Polysaccharidanteil in den Vesikeln bezogen auf das Gesamtgewicht der Vesikel in dem Bereich von 1 bis 85 Gew.-% liegt.

4. Formulierung gemäß einem der vorangehenden Ansprüche, **dadurch gekennzeichnet, dass** die Summe der Anteile aller Ladungsgeber in den Vesikeln bezogen auf das Gesamtgewicht des UV-Schutzmittels in dem Bereich von 0,01 bis 10 Gew.-% liegt.

5. Formulierung gemäß einem der vorangehenden Ansprüche, **dadurch gekennzeichnet, dass** die hydrophobierten Polysaccharide, aus denen die Vesikel aufgebaut sind, ein Polysaccharid-Grundgerüst aus Polyglucose oder Polyfructose aufweisen.

6. Formulierung gemäß einem der vorangehenden Ansprüche, **dadurch gekennzeichnet, dass** das liposomale Trägersystem aus Vesikeln besteht, die aus Lipiden aufgebaut sind und eine Teilchengröße von 10 bis 1000 nm aufweisen.

7. Formulierung gemäß einem der vorangehenden Ansprüche, **dadurch gekennzeichnet, dass** die Lipide, aus denen die liposomalen Vesikel aufgebaut sind, unter Ceramiden, Phospholipiden, Glycosphingolipiden und/oder Di-Acylglycosiden ausgewählt sind.

8. Formulierung gemäß einem der vorangehenden Ansprüche, **dadurch gekennzeichnet, dass** der Anteil der Lipide, aus denen die liposomalen Vesikel aufgebaut sind, bezogen auf die gesamte Formulierung 1 bis 20 Gew.-% beträgt.

9. Formulierung gemäß einem der vorangehenden Ansprüche, **dadurch gekennzeichnet, dass** der wenigstens eine antioxidative Wirkstoff ausgewählt ist aus der Gruppe der Vitamine.

10. Formulierung gemäß einem der Ansprüche 1 bis 8, **dadurch gekennzeichnet, dass** der wenigstens eine antioxidative Wirkstoff ausgewählt ist unter Ascorbinsäure, Tocopherol (Vitamin E) Tert-butyl-4-methoxyphenol, 2,6-Di-tert-butyl-p-kresol, 4-Butylresorcinol, 4-(1-Phenylethyl)resorcinol, Ubichinol, 6-all-*trans*-Decaprenyl-2,3-dimethoxy-5-methyl-1,4-benzochinon, 6-(10-Hydroxydecyl)-2,3-dimethoxy-5-methyl-1,4-benzochinon, Vanillinsäure, Gallussäure, Protocatechusäure, Kaffeesäure und p-Cumarsäure sowie deren Ester mit C₂-C₁₈-Alkoholen bzw. -Fettalkoholen, Resveratrol, Ellagsäure und Rosmarinsäure, Chalkonen, Flavonen, Flavonolen, Flavanolen, Flavanonen, Flavanonolen, Isoflavonen, Anthocyanen und Auronen polyphenolreichen Pflanzenextrakten, antioxidativ wirksamen Enzymen, Glutathion und Alkali- und Erdalkalisulfiten und -disulfiten und Alkalibisulfiten.

11. Formulierung gemäß einem der vorangehenden Ansprüche, **dadurch gekennzeichnet, dass** der Anteil des wenigstens einen antioxidativen Wirkstoffs bezogen auf die gesamte Formulierung 0,01 - 20 Gew.-% beträgt.

12. Formulierung gemäß einem der vorangehenden Ansprüche, **dadurch gekennzeichnet, dass** die wenigstens eine UV-Filtersubstanz ausgewählt ist unter 3-Benzylidencampher, 4-Methylbenzylidencampher, Benzophenon-1, Benzophenon-2, Benzophenon-3, Benzophenon-4, Benzophenon-5, Benzophenon-6, Benzophenon-9, Benzylidencamphersulfonsäure, Bis-Ethylhexyloxyphenolmethoxyphenyltriazin, Butylmethoxydibenzoylmethan, Campherbenzalkoniummethosulfat, Diethylaminohydroxybenzoylhexylbenzoat, Diethylhexylbutamidotriazon, Dinatriumphenyldibenzimidazoltetrasulfonat, Drometrizoltrisiloxan, Ethylhexyldimethylpaba, Ethylhexylmethoxycinnamat, Ethylhexylsalicylat, Ethylhexyltriazon, Homosalat, Isoamyl-p-methoxycinnamat, Methylen-bis-benzotriazolyltetramethylbutylphenol, Octocrylen, PEG-25-Paba, Phenylbenzimidazolsulfonsäure, Polyacrylamidomethylbenzylidencampher, Polysilicon-15, Kalium-phenylbenzimidazolsulfonat, Natriummangoseedat, Natriumphenylbenzimidazolsulfonat, Tea-Phenylbenzimidazolsulfonat, Terephthalylidendicamphersulfonsäure, Ferulasäure, Cinoxat, Diisopropylmethylcinnamat, 4-(2-Beta-Glucopyranosiloxy)propoxy-2-hydroxybenzophenon, Glycerylethylhexanoatdimethoxycinnamat, Isopentyltrimethoxycinnamattrisiloxan.

13. Formulierung gemäß einem der vorangehenden Ansprüche, **dadurch gekennzeichnet, dass** es zusätzlich einen oder mehrere Hilfsstoffe umfasst.

14. Formulierung gemäß Anspruch 13, **dadurch gekennzeichnet, dass** der Hilfsstoffanteil in den Vesikeln bezogen auf das Gesamtgewicht des UV-Schutzmittels in dem Bereich von 0,01 bis 10 Gew.-% liegt.

## Claims

1. Cosmetic or pharmaceutical formulation comprising a UV protection composition and additionally an antioxidative active ingredient, **characterized in that** the at least one antioxidative active ingredient is encapsulated in a liposomal carrier system that penetrates the skin or hair, and the UV protection composition for topical application to the skin or hair comprises at least one UV filter substance which is encapsulated in a vesicular carrier system that does not penetrate the skin or hair, wherein the at least one UV filter substance has an n-octanol-water partition coefficient K_{ow} at 25°C of greater than 1 and the vesicular carrier system consists of vesicles that are made up of hydrophobized polysaccharides and have a particle size of 10 to 1000 nm and a positive surface charge with a zeta potential in the range of 1 to 150 mV, wherein the hydrophobized polysaccharides of which the vesicles are composed are hydrophobized **in that** the C₃₋₂₂-alkyl groups are bonded to the hydroxyl groups of the polysaccharide via alkyl-ether bonds or via alkyl-urethane bonds or are bonded to the polysaccharide base structure via a linker, and wherein the positive surface charge is produced **in that** the vesicles, in addition to the polysaccharides of which the vesicles are composed, have positively charged molecules as charge providers, wherein these charge providers are selected from:
a) N-stearyl-N,N-dimethyl-N-(2-hydroxy-3-panthenyl)propylammonium salts of the formula (I), 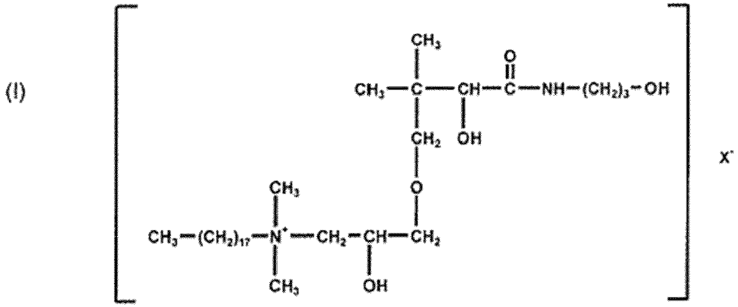
b) positively charged quaternary sugar derivatives of the formula (II) 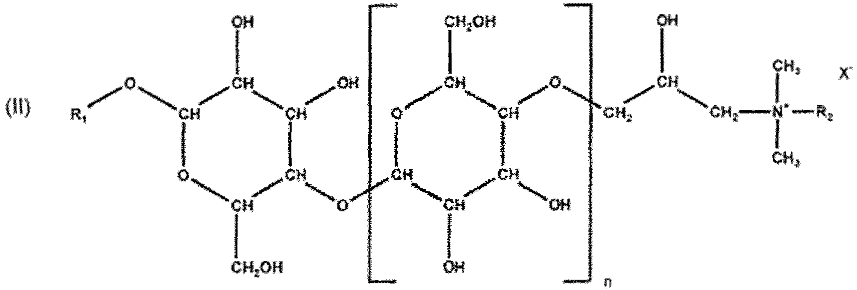 wherein R₁ and R₂ are alkyl radicals,
c) positively charged phospholipids, selected from diester and triester phosphatides of the formula (III), 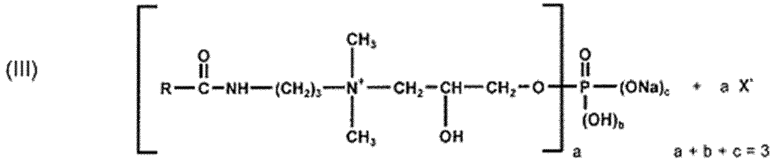 wherein the radical R is an unbranched or branched alkyl radical and/or alkenyl radical having C₇-C₂₅ carbon atoms,
d) positively charged N-(3-alkylamido)propyl-N,N-dimethyl-N-(2,3-dihydroxypropyl)ammonium salts and N-(3-alkenylamido) propyl-N, N-dimethyl-N-(2,3-dihydroxy-propyl) ammonium salts of the formula (IV) 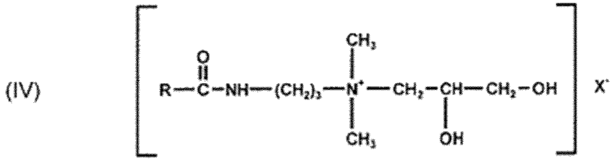 and also N-(3-ricinylamido)propyl-N,N-dimethyl-N-(2,3-dihyroxypropyl)ammonium salts,
e) positively charged quaternary N,N,N-trimethyl-N-(2-hydroxy-3-"R-ether"-propyl)ammonium salts of the formula (V) 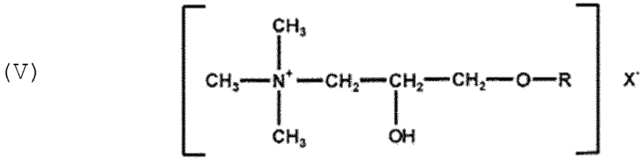
wherein the radical R is hyaluronic acid, xanthan gum or trehalose,
or combinations thereof,
and wherein the vesicles for the formation of the positive surface charge, in addition to the charge providers specified above, optionally comprise
f) one or more positively charged quaternary C₁₂-C₂₂-alkyltrimethylammonium salts (or fatty acid trimonium salts of the formula 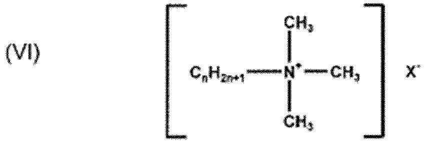 wherein X⁻ in the formulae (I) to (VI) specified above is a cosmetically or pharmaceutically compatible organic or inorganic anion.

2. Formulation according to any of the preceding claims, **characterized in that** the proportion of UV filter substance in the vesicles, based on the total weight of the UV protection composition, is in the range of 1 to 65% by weight.

3. Formulation according to any of the preceding claims, **characterized in that** the proportion of polysaccharide in the vesicles, based on the total weight of the vesicles, is in the range of 1 to 85% by weight.

4. Formulation according to any of the preceding claims, **characterized in that** the sum total of the proportions of all charge providers in the vesicles, based on the total weight of the UV protection composition, is in the range of 0.01 to 10% by weight.

5. Formulation according to any of the preceding claims, **characterized in that** the hydrophobized polysaccharides, of which the vesicles are composed, comprise a polysaccharide base structure composed of polyglucose or polyfructose.

6. Formulation according to any of the preceding claims, **characterized in that** the liposomal carrier system consists of vesicles which are made up of lipids and have a particle size of 10 to 1000 nm.

7. Formulation according to any of the preceding claims, **characterized in that** the lipids of which the liposomal vesicles are composed are selected from ceramides, phospholipids, glycosphingolipids and/or diacylglycosides.

8. Formulation according to any of the preceding claims, **characterized in that** the proportion of lipids, of which the liposomal vesicles are composed, is 1 to 20% by weight, based on the total formulation.

9. Formulation according to any of the preceding claims, **characterized in that** the at least one antioxidative active ingredient is selected from the group of vitamins.

10. Formulation according to any of Claims 1 to 8, **characterized in that** the at least one antioxidative active ingredient is selected from ascorbic acid, tocopherol (vitamin E), tert-butyl-4-methoxyphenol, 2,6-di-tert-butyl-p-cresol, 4-butylresorcinol, 4-(1-phenylethyl)resorcinol, ubiquinol, 6-all-trans-decaprenyl-2,3-dimethoxy-5-methyl-1,4-benzoquinone, 6-(10-hydroxydecyl)-2,3-dimethoxy-5-methyl-1,4-benzoquinone, vanillic acid, gallic acid, protocatechuic acid, caffeic acid and p-cumaric acid and also esters thereof with C₂-C₁₈ alcohols or C₂-C₁₈-fatty alcohols, resveratrol, ellagic acid and rosmarinic acid, chalcones, flavones, flavonols, flavanols, flavanones, flavanonols, isoflavones, anthocyanins and aurones, polyphenol-rich plant extracts, antioxidative enzymes, glutathione and alkali metal and alkaline earth metal sulfites and disulfites and alkali metal bisulfites.

11. Formulation according to any of the preceding claims, **characterized in that** the proportion of the at least one antioxidative active ingredient is 0.01 to 20% by weight, based on the total formulation.

12. Formulation according to any of the preceding claims, **characterized in that** the at least one UV filter substance is selected from 3-benzylidene camphor, 4-methylbenzylidene camphor, benzophenone-1, benzophenone-2, benzophenone-3, benzophenone-4, benzophenone-5, benzophenone-6, benzophenone-9, benzylidene camphor sulfonic acid, bis-ethylhexyloxyphenol methoxyphenyl triazine, butyl methoxydibenzoylmethane, camphor benzalkonium methosulfate, diethylamino hydroxybenzoyl hexyl benzoate, diethylhexylbutamidotriazone, disodium phenyl dibenzimidazole tetrasulfonate, drometrizole trisiloxane, ethylhexyl dimethyl PABA, ethylhexyl methoxycinnamate, ethylhexyl salicylate, ethylhexyl triazone, homosalate, isoamyl p-methoxycinnamate, methylene bis-benzotriazolyl tetramethylbutylphenol, octocrylene, PEG-25 PABA, phenylbenzimidazole sulfonic acid, polyacrylamidomethylbenzylidene camphor, polysilicone-15, potassium phenylbenzimidazole sulfonate, sodium mangoseedate, sodium phenylbenzimidazole sulfonate, TEA-phenylbenzimidazole sulfonate, terephthalylidene dicamphor sulfonic acid, ferulic acid, cinoxate, diisopropyl methylcinnamate, 4-(2-beta-glucopyranosiloxy)propoxy-2-hydroxybenzophenone, glyceryl ethylhexanoate dimethoxycinnamate, isopentyl trimethoxycinnamate trisiloxane.

13. Formulation according to any of the preceding claims, **characterized in that** said formulation comprises in addition one or more auxiliaries.

14. Formulation according to Claim 13, charcaterized in that the proportion of auxiliaries in the vesicles is in the range of 0.01 to 10% by weight, based on the total weight of the UV protection composition.

## Revendications

1. Composition cosmétique ou pharmaceutique, qui contient un protecteur anti-UV et en outre une substance active antioxydante, **caractérisée en ce que** ladite au moins une substance active antioxydante est encapsulée dans un système véhicule liposomal pénétrant dans la peau ou les cheveux et le protecteur anti-UV comprend pour l'application topique sur la peau ou les cheveux au moins une substance filtrant les UV qui est encapsulée dans un système véhicule vésiculaire ne pénétrant pas dans la peau ou les cheveux, dans laquelle ladite au moins une substance filtrant les UV présente à 25 °C un coefficient de partage n-octanol-eau *K_{ow}* qui est supérieur à 1 et le système véhicule vésiculaire consiste en des vésicules qui sont constituées de polysaccharides rendus hydrophobes et présentent une taille de particule de 10 à 1 000 nm ainsi qu'une tension superficielle positive avec un potentiel zêta dans la plage de 1 à 150 mV, dans laquelle les polysaccharides rendus hydrophobes, à base desquels sont constituées les vésicules, sont rendus hydrophobes par le fait que les groupes alkyle en C₃-C₂₂ sont liés aux groupes hydroxy du polysaccharide par les liaisons alkyle-éther ou par des liaisons alkyle-uréthane ou sont liés au squelette de base du polysaccharide par un segment de liaison, et dans laquelle la charge superficielle positive est réalisée par le fait que les vésicules comportent, en plus des polysaccharides à base desquels sont constituées les vésicules, des molécules chargées positivement en tant que donneurs de charge, ces donneurs de charge étant choisis parmi :
a) des sels de N-stéaryl-N,N-diméthyl-N-(2-hydroxy-3-panthényl)propyl-ammonium correspondant à la formule (I) ; 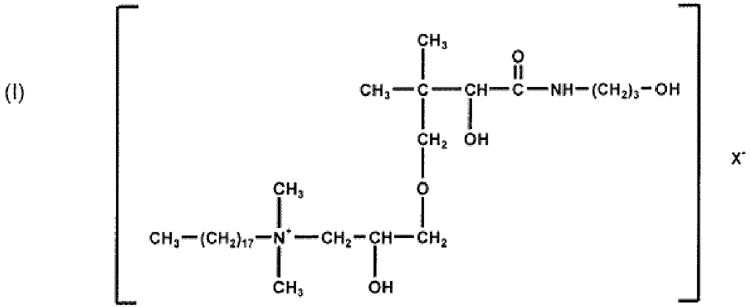
b) des dérivés de sucres quaternaires chargés positivement, correspondant à la formule (II) 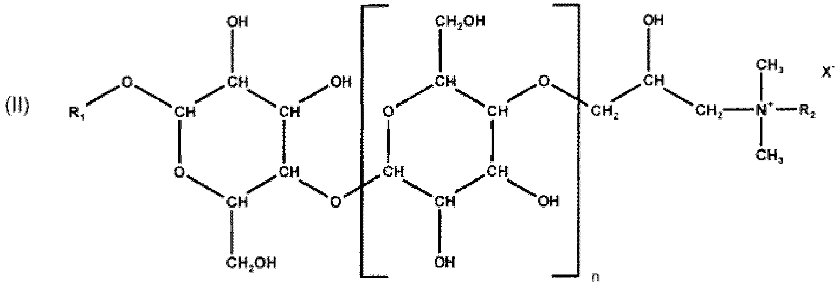 dans laquelle R₁ et R₂ sont des radicaux alkyle,
c) des phospholipides chargés positivement, choisis parmi des diester- et triesterphosphatides correspondant à la formule (III), 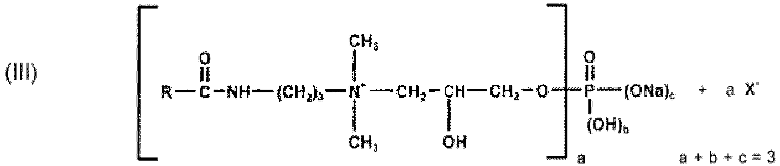 dans laquelle le radical R est un radical alkyle et/ou un radical alcényle, ramifié(s) ou non ramifié(s), ayant de 7 à 25 atomes de carbone,
d) des sels de N-(3-alkylamido)propyl-N,N-diméthyl-N-(2,3-dihydroxypropyl)-ammonium et sels de N-(3-alcénylamido)propyl-N,N-diméthyl-N-(2,3-dihydroxypropyl)-ammonium, chargés positivement, correspondant à la formule (IV) 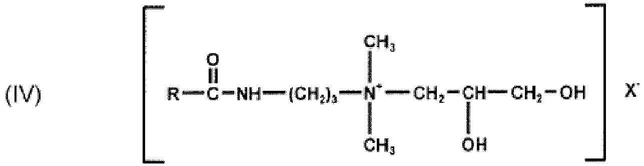 ainsi que des sels de N-(3-ricinylamido)propyl-N,N-diméthyl-N-(2,3-dihydroxypropyl)-ammonium,
e) des sels de N,N,N-triméthyl-N-(2-hydroxy-3-« *R-éther* »-propyl)-ammonium chargés positivement, correspondant à la formule (V) 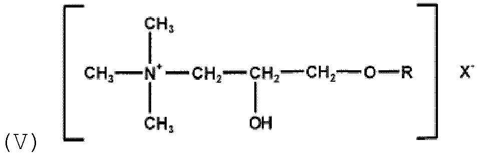 dans laquelle le radical R représente l'acide hyaluronique, la gomme xanthane ou le tréhalose,
ou des associations de ceux-ci,
et dans laquelle pour la création de la charge superficielle positive les vésicules comportent en option, en plus des donneurs de charge indiqués, encore
f) un ou plusieurs sels d'alkyl (C₁₂-C₂₂) - trilméthylammonium (ou sels d'acide grastrimonium) quaternaires chargés positivement, correspondant à la formule 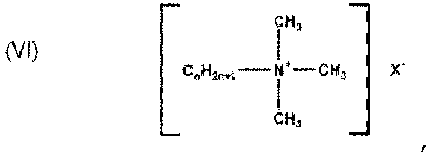 dans les formules (I) à (VI) indiquées plus haut X⁻ étant un anion organique ou inorganique cosmétiquement ou pharmaceutiquement acceptable.

2. Composition selon l'une quelconque des revendications précédentes, **caractérisée en ce que** la proportion de la substance filtrant les UV dans les vésicules, par rapport au poids total du protecteur anti-UV, se situe dans la plage de 1 à 65 % en poids.

3. Composition selon l'une quelconque des revendications précédentes, **caractérisée en ce que** la proportion de polysaccharide dans les vésicules, par rapport au poids total des vésicules, se situe dans la plage de 1 à 85 % en poids.

4. Composition selon l'une quelconque des revendications précédentes, **caractérisée en ce que** la somme des proportions de tous les donneurs de charge dans les vésicules, par rapport au poids total du protecteur anti-UV, se situe dans la plage de 0,01 à 10 % en poids.

5. Composition selon l'une quelconque des revendications précédentes, **caractérisée en ce que** les polysaccharides rendus hydrophobes, à base desquels sont constituées les vésicules, comportent un squelette de base de polysaccharide à base de polyglucose ou polyfructose.

6. Composition selon l'une quelconque des revendications précédentes, **caractérisée en ce que** le système véhicule liposomal consiste en des vésicules qui sont constitués de lipides et présentent une taille de particule de 10 à 1 000 nm.

7. Composition selon l'une quelconque des revendications précédentes, **caractérisée en ce que** les lipides, à base desquels sont constituées les vésicules liposomales, sont choisis parmi les céramides, les phospholipides, les glycosphingolipides et/ou les diacylglycosides.

8. Composition selon l'une quelconque des revendications précédentes, **caractérisée en ce que** la proportion des lipides, à base desquels sont constituées les vésicules liposomales, vaut de 1 à 20 % en poids par rapport à la composition totale.

9. Composition selon l'une quelconque des revendications précédentes, **caractérisée en ce que** ladite au moins une substance active antioxydante est choisie dans le groupe des vitamines.

10. Composition selon l'une quelconque des revendications 1 à 8, **caractérisée en ce que** ladite au moins une substance active antioxydante est choisie parmi l'acide ascorbique, le tocophérol (vitamine E), le tert-butyl-4-méthoxyphénol, le 2,6-di-tert-butyl-p-crésol, le 4-butylrésorcinol, le 4-(1phényléthyl)-résorcinol, l'ubiquinol, la 6-tout-trans-décaprényl-2,3-diméthoxy-5-méthyl-1,4-benzoquinone, la 6-(10-hydroxydécyl)-2,3-diméthoxy-5-méthyl-1,4-benzoquinone, l'acide vanillique, l'acide gallique, l'acide protocatéchique, l'acide caféique et l'acide p-coumarique ainsi que leurs esters avec des alcools ou alcools gras en C₂-C₁₈, le resvératrol, l'acide ellagique et l'acide rosmarinique, les extraits de plantes riches en polyphénols chalcones, flavones, flavonols, flavanols, flavanones, flavanonols, isoflavones, anthocyanes et aurones, les enzymes à activité antioxydante, le glutathion et les sulfites et disulfites de métaux alcalins et alcalino-terreux et les bisulfites de métaux alcalins.

11. Composition selon l'une quelconque des revendications précédentes, **caractérisée en ce que** la proportion de ladite au moins une substance active antioxydante vaut de 0,01 à 20 % en poids par rapport à la composition totale.

12. Composition selon l'une quelconque des revendications précédentes, **caractérisée en ce que** ladite au moins une substance filtrant les UV est choisie parmi le 3-benzylidènecamphre, le 4-méthylbenzylidènecamphre, la benzophénone-1, la benzophénone-2, la benzophénone-3, la benzophénone-4, la benzophénone-5, la benzophénone-6, la benzophénone-9, l'acide benzylidènecamphosulfonique, la bis-éthylhexyloxyphénolméthoxyphényltriazine, le butylméthoxydibenzoylméthane, le méthosulfate de camphobenzalkonium, le benzoate de diéthylaminohydroxybenzoylhexyle, la diéthylhexylbutamidotriazone, le phenyldibenzimidazoletétrasulfonate disodique, le drométrizoltrisiloxane, l'éthylhexyldiméthylPABA, le méthoxycinnamate d'éthylhexyle, le salicylate d'éthylhexyle, l'éthylhexyltriazone, l'homosalate, le p-méthoxycinnamate d'isoamyle, le méthylène-bis-benzotriazolyltétraméthylbutylphénol, l'octocrylène, le PEG-25-PABA, l'acide phénylbenzimidazolesulfonique, le polyacrylamidométhylbenzylidènecamphre, le polysilicone-15, le phénylbenzimidazolesulfonate de potassium, le mangoseedate de sodium, le phénylbenzimidazolesulfonate de sodium, le phénylbenzimidazolesulfonate de TEA, l'acide téréphtalylidènedicamphosulfonique, l'acide férulique, le cinoxate, le cinnamate de diisopropylméthyle, la 4-(2-bêta-glucopyranosiloxy)propoxy-2-hydroxybenzophénone, l'éthylhexanoate-diméthoxycinnamate de glycéryle, l'isopentyltriméthoxycinnamate-trisiloxane.

13. Composition selon l'une quelconque des revendications précédentes, **caractérisée en ce qu'**elle comprend en outre un ou plusieurs adjuvants.

14. Composition selon la revendication 13, **caractérisée en ce que** la proportion d'adjuvant(s) dans les vésicules se situe dans la plage de 0,01 à 10 % en poids, par rapport au poids total du protecteur anti-UV.
